# EUROPEAN PATENT APPLICATION

(11) **EP 2 924 038 A1**
(43) Date of publication of application: **30.09.2015**
(21) Application number: 14161293.7
(22) Date of filing: 24.03.2014
(51) Int. Cl.: C07D 417/04, C07D 487/04

(54) **Preparation of pyrrolo[2,3-b]pyrazine and pyrrolo[2,3-b]pyridine derivatives as FGFR inhibitors**

(71) Applicant: Evotec (UK) Ltd., Abingdon, Oxfordshire OX14 4RZ (GB)
(72) Inventor: Mills, Matthew, Abingdon, Oxfordshire OX14 4RZ (GB); McCarthy, Clive, Abingdon, Oxfordshire OX14 4RZ (GB)
(74) Representative: Büchel, Edwin

(57) **Abstract**

The present invention relates to the preparation of compounds of formula (I) 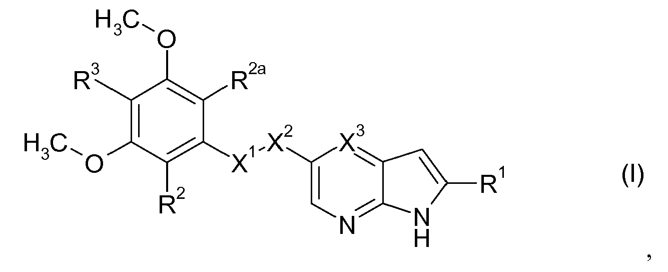 wherein R¹, R², R^{2a}, R³, X¹ to X³ have the meaning as indicated in the description and claims and to intermediate compounds used to prepare compounds of formula (I).

## Description

The present invention relates to the preparation of pyrrolo[2,3b]pyrazine and pyrrolo[2,3b]pyridine derivatives useful as FGFR inhibitors and to intermediate compounds used to prepare such compounds.

Inhibitors of the fibroblast growth factor receptor (FGFR) family are known to be useful for the treatment or prevention of proliferative disorders and dysplasia.

Some of these inhibitors and methods for their preparation are known from WO 2012/073017 Al and in unpublished international patent application with application number PCT/EP2013/069721.

Pyrrolo[2,3b]pyrazine and pyrrolo[2,3b]pyridine derivatives as FGFR inhibitors are described in unpublished international patent application with application number PCT/EP2013/069728. The compounds are prepared by reacting a dimethoxyphenyl with the central pyrrolo[2,3b]pyrazine or pyrrolo[2,3b]pyridine core in a final reaction step in order to derivatize the core on the six-membered ring of the core. However in order to also provide derivatives with regard to the substitution in the five-membered ring of the core it is highly desirable to find a synthesis strategy, where such substituents are introduced at the end of the synthesis route.

Accordingly, an object of the present invention is to provide a method for the preparation as outlined above for compounds described in unpublished international patent application with application number PCT/EP2013/069728.

This object is achieved by a method for the preparation of a compound of formula (I) 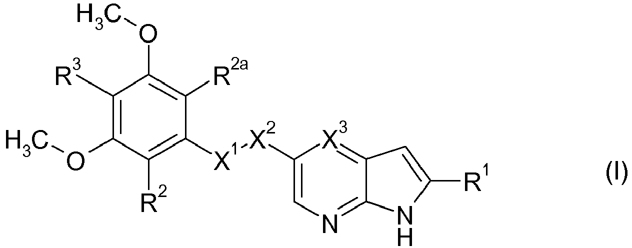 or a pharmaceutically acceptable salt thereof, wherein
X¹ is CH₂ and X² is CH₂; O; or N(R⁰), or
X¹ and X² are CH;
R⁰ is H; or C₁₋₄ alkyl, wherein C₁₋₄ alkyl is optionally substituted with one or more halogen, which are the same or different;
X³ is N; or CH;
R¹ is phenyl; naphthyl; 5 to 6 membered aromatic heterocyclyl; or 9 to 10 membered aromatic heterobicyclyl, wherein R¹ is optionally substituted with one or more R⁴, which are the same or different;
R⁴ is halogen; CN; C(O)OR⁵; OR⁵; C(O)R⁵; C(O)N(R⁵R^{5a}); S(O)₂N(R⁵R^{5a}); S(O)N(R⁵R^{5a}); S(O)₂R⁵; S(O)R⁵; N(R⁵)S(O)₂N(R^{5a}R^{5b}); SR⁵; N(R⁵R^{5a}); NO₂; OC(O)R⁵; N(R⁵)C(O)R^{5a}; N(R⁵)S(O)₂R^{5a}; N(R⁵)S(O)R^{5a}; N(R⁵)C(O)OR^{5a}; N(R⁵)C(O)N(R^{5a}R^{5b}); OC(O)N(R⁵R^{5a}); T¹; C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁶, which are the same or different;
R⁵, R^{5a}, R^{5b} are independently selected from the group consisting of H; T¹; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁶, which are the same or different;
R⁶ is halogen; CN; C(O)OR⁷; OR⁷; C(O)R⁷; C(O)N(R⁷R^{7a}); S(O)₂N(R⁷R^{7a}); S(O)N(R⁷R^{7a}); S(O)₂R⁷; S(O)R⁷; N(R⁷)S(O)₂N(R^{7a}R^{7b}); SR⁷; N(R⁷R^{7a}); NO₂; OC(O)R⁷; N(R⁷)C(O)R^{7a}; N(R⁷)SO₂R^{7a}; N(R⁷)S(O)R^{7a}; N(R⁷)C(O)N(R^{7a}R^{7b}); N(R⁷)C(O)OR^{7a}; OC(O)N(R⁷R^{7a}); or T¹; R⁷, R^{7a}, R^{7b} are independently selected from the group consisting of H; T¹; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
T¹ is phenyl; C₃₋₇ cycloalkyl; or 3 to 7 membered heterocyclyl, wherein T¹ is optionally substituted with one or more R⁸, which are the same or different;
R⁸ is halogen; CN; C(O)OR⁹; OR⁹; C(O)R⁹; C(O)N(R⁹R^{9a}); S(O)₂N(R⁹R^{9a}); S(O)N(R⁹R^{9a}); S(O)₂R⁹; S(O)R⁹; N(R⁹)S(O)₂N(R^{9a}R^{9b}); SR⁹; N(R⁹R^{9a}); NO₂; OC(O)R⁹; N(R⁹)C(O)R^{9a}; N(R⁹)S(O)₂R^{9a}; N(R⁹)S(O)R^{9a}; N(R⁹)C(O)OR^{9a}; N(R⁹)C(O)N(R^{9a}R^{9b}); OC(O)N(R⁹R^{9a}); oxo (=O), where the ring is at least partially saturated; C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more halogen, which are the same or different;
R⁹, R^{9a}, R^{9b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
R²; R^{2a}; R³ are independently selected from the group consisting of H; and halogen,
wherein the method comprises the step of
   (a₀) reacting a compound of formula (Ia) 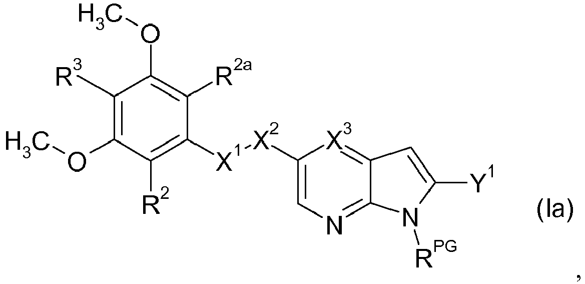 with a compound of formula R¹-Y² (Ib) in a coupling reaction, wherein X¹, X², X³, R², R^{2a}, R³ have the above meaning, R^{PG} is H or PG, wherein PG is a suitable protective group and Y¹, Y² are capable to react in a metal catalyzed coupling reaction forming carbon-carbon bond; and subsequent deprotection in order to remove PG, if present, to yield a compound of formula (I).

Surprisingly a convenient route has been found comprising the metal coupling reaction as a last step in order to provide compounds of formula (I). Especially in case that Y¹ is I it was surprisingly found that selective iodination is possible.

In case a variable or substituent can be selected from a group of different variants and such variable or substituent occurs more than once the respective variants can be the same or different.

Within the meaning of the present invention the terms are used as follows:
The term "optionally substituted" means unsubstituted or substituted. Generally -but not limited to-, "one or more substituents" means one, two or three, preferably one or two substituents and more preferably one substituent. Generally these substituents can be the same or different.

"Alkyl" means a straight-chain or branched hydrocarbon chain. Each hydrogen of an alkyl carbon may be replaced by a substituent as further specified.

"Alkenyl" means a straight-chain or branched hydrocarbon chain that contains at least one carbon-carbon double bond. Each hydrogen of an alkenyl carbon may be replaced by a substituent as further specified.

"Alkynyl" means a straight-chain or branched hydrocarbon chain that contains at least one carbon-carbon triple bond. Each hydrogen of an alkynyl carbon may be replaced by a substituent as further specified.

"C₁₋₃ alkyl" means an alkyl chain having 1 - 3 carbon atoms, e.g. if present at the end of a molecule: methyl, ethyl, n-propyl, isopropyl, or e.g. -CH₂-, -CH₂-CH₂-, -CH(CH₃)-, -CH₂-CH₂-CH₂-, -CH(C₂H₅)-, -C(CH₃)₂-, when two moieties of a molecule are linked by the alkyl group. Each hydrogen of a C₁₋₃ alkyl carbon may be replaced by a substituent as further specified.

"C₁₋₄ alkyl" means an alkyl chain having 1 - 4 carbon atoms, e.g. if present at the end of a molecule: methyl, ethyl, n-propyl, isopropyl, n-butyl, isobutyl, sec-butyl, tert-butyl, or e.g.-CH₂-, -CH₂-CH₂-, -CH(CH₃)-, -CH₂-CH₂-CH₂-, -CH(C₂H₅)-, -C(CH₃)₂-, when two moieties of a molecule are linked by the alkyl group. Each hydrogen of a C₁₋₄ alkyl carbon may be replaced by a substituent as further specified.

"C₁₋₆ alkyl" means an alkyl chain having 1 - 6 carbon atoms, e.g. if present at the end of a molecule: C₁₋₄ alkyl, methyl, ethyl, n-propyl, isopropyl, n-butyl, isobutyl, sec-butyl; tert-butyl, n-pentyl, n-hexyl, or e.g. -CH₂-, -CH₂-CH₂-, -CH(CH₃)-, -CH₂-CH₂-CH₂-, -CH(C₂H₅)-,-C(CH₃)₂-, when two moieties of a molecule are linked by the alkyl group. Each hydrogen of a C₁₋₆ alkyl carbon may be replaced by a substituent as further specified.

"C₂₋₆ alkenyl" means an alkenyl chain having 2 to 6 carbon atoms, e.g. if present at the end of a molecule: -CH=CH₂, -CH=CH-CH₃, -CH₂-CH=CH₂, -CH=CH-CH₂-CH₃, -CH=CH-CH=CH₂, or e.g. -CH=CH-, when two moieties of a molecule are linked by the alkenyl group. Each hydrogen of a C₂₋₆ alkenyl carbon may be replaced by a substituent as further specified.

"C₂₋₆ alkynyl" means an alkynyl chain having 2 to 6 carbon atoms, e.g. if present at the end of a molecule: -C≡CH, -CH₂-C≡CH, CH₂-CH₂-C≡CH, CH₂-C≡C-CH₃, or e.g. -C=C- when two moieties of a molecule are linked by the alkynyl group. Each hydrogen of a C₂₋₆ alkynyl carbon may be replaced by a substituent as further specified.

"C₃₋₇ cycloalkyl" or "C₃₋₇ cycloalkyl ring" means a cyclic alkyl chain having 3 - 7 carbon atoms, e.g. cyclopropyl, cyclobutyl, cyclopentyl, cyclohexyl, cyclohexenyl, cycloheptyl. Preferably, cycloalkyl refers to cyclopropyl, cyclobutyl, cyclopentyl, cyclohexyl, or cycloheptyl. Each hydrogen of a cycloalkyl carbon may be replaced by a substituent as further specified herein. The term "C₃₋₅ cycloalkyl" or "C₃₋₅ cycloalkyl ring" is defined accordingly.

"Halogen" means fluoro, chloro, bromo or iodo. It is generally preferred that halogen is fluoro or chloro.

"3 to 7 membered heterocyclyl" or "3 to 7 membered heterocycle" means a ring with 3, 4, 5, 6 or 7 ring atoms that may contain up to the maximum number of double bonds (aromatic or non-aromatic ring which is fully, partially or un-saturated) wherein at least one ring atom up to 4 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a 3 to 7 membered heterocycles are aziridine, azetidine, oxetane, thietane, furan, thiophene, pyrrole, pyrroline, imidazole, imidazoline, pyrazole, pyrazoline, oxazole, oxazoline, isoxazole, isoxazoline, thiazole, thiazoline, isothiazole, isothiazoline, thiadiazole, thiadiazoline, tetrahydrofuran, tetrahydrothiophene, pyrrolidine, imidazolidine, pyrazolidine, oxazolidine, isoxazolidine, thiazolidine, isothiazolidine, thiadiazolidine, sulfolane, pyran, dihydropyran, tetrahydropyran, imidazolidine, pyridine, pyridazine, pyrazine, pyrimidine, piperazine, piperidine, morpholine, tetrazole, triazole, triazolidine, tetrazolidine, diazepane, azepine or homopiperazine. The term "5 to 6 membered heterocyclyl" or "5 to 6 membered heterocycle" is defined accordingly.

"Saturated 4 to 7 membered heterocyclyl" or "saturated 4 to 7 membered heterocycle" means fully saturated "4 to 7 membered heterocyclyl" or "4 to 7 membered heterocycle".

"4 to 7 membered at least partly saturated heterocyclyl" or "4 to 7 membered at least partly saturated heterocycle" means an at least partly saturated "4 to 7 membered heterocyclyl" or "4 to 7 membered heterocycle".

"5 to 6 membered aromatic heterocyclyl" or "5 to 6 membered aromatic heterocycle" means a heterocycle derived from cyclopentadienyl or benzene, where at least one carbon atom is replaced by a heteroatom selected from the group consisting of sulfur (including -S(O)-, - S(O)₂-), oxygen and nitrogen (including =N(O)-). Examples for such heterocycles are furan, thiophene, pyrrole, imidazole, pyrazole, oxazole, isoxazole, thiazole, isothiazole, thiadiazole, triazole, tetrazole, pyridine, pyrimidine, pyridazine, pyrazine, triazine.

"5 membered aromatic heterocyclyl" or "5 membered aromatic heterocycle" means a heterocycle derived from cyclopentadienyl, where at least one carbon atom is replaced by a heteroatom selected from the group consisting of sulfur (including -S(O)-, -S(O)₂-), oxygen and nitrogen (including =N(O)-). Examples for such heterocycles are furan, thiophene, pyrrole, imidazole, pyrazole, oxazole, isoxazole, thiazole, isothiazole, thiadiazole, triazole, tetrazole.

"7 to 11 membered heterobicyclyl" or "7 to 11 membered heterobicycle" means a heterocyclic system of two rings with 7 to 11 ring atoms, where at least one ring atom is shared by both rings and that may contain up to the maximum number of double bonds (aromatic or non-aromatic ring which is fully, partially or un-saturated) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a 7 to 11 membered heterobicycle are indole, indoline, benzofuran, benzothiophene, benzoxazole, benzisoxazole, benzothiazole, benzisothiazole, benzimidazole, benzimidazoline, quinoline, quinazoline, dihydroquinazoline, quinoline, dihydroquinoline, tetrahydroquinoline, decahydroquinoline, isoquinoline, decahydroisoquinoline, tetrahydroisoquinoline, dihydroisoquinoline, benzazepine, purine or pteridine. The term 7 to 11 membered heterobicycle also includes spiro structures of two rings like 6-oxa-2-azaspiro[3,4]octane, 2-oxa-6-azaspiro[3.3]heptan-6-yl or 2,6-diazaspiro[3.3]heptan-6-yl or bridged heterocycles like 8-aza-bicyclo[3.2.1]octane or 2,5-diazabicyclo[2.2.2]octan-2-yl or 3,8-diazabicyclo[3.2.1]octane.

"Saturated 7 to 11 membered heterobicyclyl" or "saturated 7 to 11 membered heterobicycle" means fully saturated 7 to 11 membered heterobicyclyl or 7 to 11 membered heterobicycle.

"7 to 11 membered at least partly saturated heterobicyclyl" or "7 to 11 membered at least partly saturated heterobicycle" means an at least partly saturated "7 to 11 membered heterobicyclyl" or "7 to 11 membered heterobicycle".

"9 to 11 membered aromatic heterobicyclyl" or "9 to 11 membered aromatic heterobicycle" means a heterocyclic system of two rings, wherein at least one ring is aromatic and wherein the heterocyclic ring system has 9 to 11 ring atoms, where two ring atoms are shared by both rings and that may contain up to the maximum number of double bonds (fully or partially aromatic) wherein at least one ring atom up to 6 ring atoms are replaced by a heteroatom selected from the group consisting of sulfur (including -S(O)-, -S(O)₂-), oxygen and nitrogen (including =N(O)-) and wherein the ring is linked to the rest of the molecule via a carbon or nitrogen atom. Examples for a 9 to 11 membered aromatic heterobicycle are indole, indoline, benzofuran, benzothiophene, benzoxazole, benzisoxazole, benzothiazole, benzisothiazole, benzimidazole, benzimidazo line, quinoline, quinazoline, dihydroquinazo line, dihydroquino line, tetrahydroquinoline, isoquinoline, tetrahydroisoquinoline, dihydroisoquinoline, benzazepine, purine or pteridine. The terms "9 to 10 membered aromatic heterobicyclyl" or "9 to 10 membered aromatic heterobicycle" are defined accordingly.

In general Y¹ and Y² serve as leaving groups in order to form the carbon-carbon bond.

In a preferred embodiment of the present invention Y¹ and Y² are selected in that
(i) Y¹ is B(OR^{A})₂ and Y² is Br, I or OS(O)₂CF₃; or Y¹ is Br, I and Y² is B(OR^{A})₂; or
(ii) Y¹ is SnR^{B}₃ and Y² is Br, I or OS(O)₂CF₃; or Y¹ is Br, I and Y² is SnR^{B}₃; or
(iii) Y¹ is ZnBr or ZnI and Y² is Br, I or OS(O)₂CF₃; or Y¹ is Br, I and Y² is ZnBr or ZnI, wherein each R^{A} is independently H, C₁₋₄ alkyl or (OR^{A})₂ is pinacolate and each R^{B} is C₁₋₄ alkyl.

A more preferred embodiment is option (i). Preferably, the protective group PG is phenylsulfonyl or 4-methylphenylsulfonyl. Thus deprotection can be accomplished by subjecting the protected compound to an alkaline liquid, e.g. NaOH/methanol. In reaction step (a₀) the compound of formula (Ia) can be used in protected form (R^{PG} = PG) or unprotected form (R^{PG} = H). In case the compound of formula (Ia) is protected, the reaction step (a₀) can be carried out so that deprotection is achieved during said reaction step or afterwards. In the second case deprotection can be obtained with or without isolation the intermediate compound. Also preferred is a method of the invention, wherein Y¹ is I and/or Y² is B(OR^{A})₂.

Resulting compounds in case step (a₀) is carried out before deprotection are compounds of formula (Ic) compound of formula (Ic) 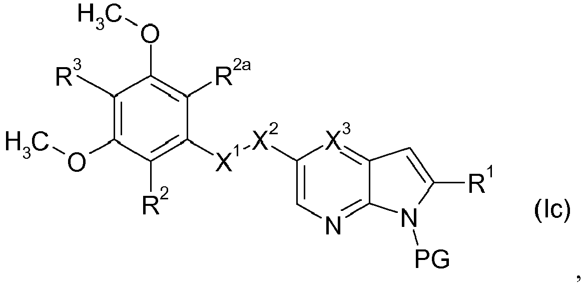 wherein R¹, R², R^{2a}, R³, X¹, X², X³, PG have the above meaning.

Based on the options for Y¹ and Y² different metal catalyzed coupling reactions are known in the state of the art. Typically, the metal that can be used is Pd⁰. Examples of conditions used in the coupling reactions are reviewed in Journal of Organic Chemistry, 2012, Volume 77, Issue 12, pages 5221-5223.

For option (i) Y¹ and Y² are selected to represent suitable leaving groups which are known from the Suzuki coupling. Accordingly, Y¹ is B(OR^{A})₂ and Y² is Br, I or OS(O)₂CF₃; or Y¹ is Br, I and Y² is B(OR^{A})₂, wherein each R^{A} is independently H, C₁₋₄ alkyl or (OR^{A})₂ is pinacolate, i.e. tetramethyl-1,3,2-dioxaborolan-2-yl, which is preferred as well as methyl for each R^{A}. Preferably, Y¹ is I.

For option (ii) Y¹ and Y² are selected to represent suitable leaving groups which are known from the Stille reaction. Accordingly, Y¹ is SnR^{B}₃ and Y² is Br, I or OS(O)₂CF₃; or Y¹ is Br, I and Y² is SnR^{B}₃, wherein each R^{B} is C₁₋₄ alkyl. Typical representatives for SnR^{B}₃ are the respective trimethyl or tri butyl derivatives.

For option (iii) Y¹ and Y² are selected to represent suitable leaving groups which are known from the Negishi reaction. Accordingly, Y¹ is ZnBr or ZnI and Y² is Br, I or OS(O)₂CF₃; or Y¹ is Br, I and Y² is ZnBr or ZnI. Typically, the organic zinc compounds are only prepared in situ before the coupling reaction. Thus for the method of the present invention the starting material can be provided in isolated form or only in situ prepared before reaction step (a₀).

For the method of the present invention preferred compounds of formula (I), formula (Ia) and (Ib) respectively, are those compounds in which one or more of the residues contained therein have the meanings given below, with all combinations of preferred substituent definitions being a subject of the present invention. With respect to all preferred compounds of the formula (I) the present invention also includes all tautomeric and stereoisomeric forms and mixtures thereof in all ratios, and their pharmaceutically acceptable salts.

In preferred embodiments of the present invention, the substituents mentioned below independently have the following meaning. Hence, one or more of these substituents can have the preferred or more preferred meanings given below.

In one embodiment of the present invention, X³ is N. In another embodiment X³ is CH.

Preferably, R¹ is phenyl; or pyridyl, wherein R¹ is unsubstituted or substituted with one or more R⁴, which are the same or different.

Preferably, R¹ is substituted with one or more R⁴, which are the same or different.

Preferably, R¹ in formula (I) is selected to give formula (IA) 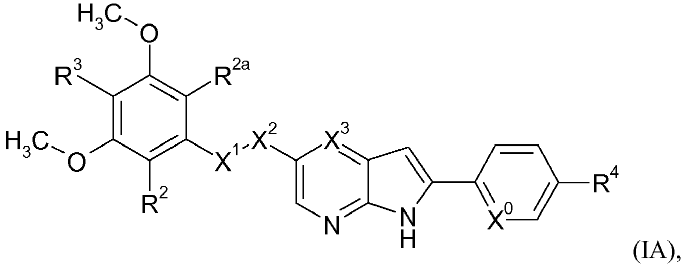 wherein X⁰ is N; or CH; and X¹, X², X³, R², R^{2a}, R³, R⁴ have the meaning as indicated above.

In one embodiment, X⁰ is CH. In another embodiment, X⁰ is N.

Preferably, R⁴ is T¹; or C₁₋₆ alkyl, substituted with T¹. More preferably, R⁴ is T¹; or C₁₋₃ alkyl, substituted with T¹. In particular, R⁴ is T¹; or CH₂-T¹.

Preferably, T¹ is saturated 4 to 7 membered heterocyclyl, wherein T¹ is unsubstituted or substituted with one or more R⁸, which are the same or different. More preferably, T¹ is piperazinyl; piperidinyl; or morpholinyl, wherein T¹ is unsubstituted or substituted with one or more R⁸, which are the same or different.

Preferably, R⁸ is C₁₋₆ alkyl; or oxo, where the ring is at least partially saturated. More preferably, R⁸ is C₁₋₃ alkyl; or oxo, where the ring is at least partially saturated. Even more preferably, R⁸ is C₁₋₆ alkyl. Even more preferably, R⁸ is C₁₋₃ alkyl.

Preferably, R⁴ is piperazin-1-ylmethyl; 4-methylpiperazin-1-yl; 4-ethylpiperazin-1-yl; morpholin-4-yl; (4-methylpiperazin-1-yl)methyl; (4-ethylpiperazin-1-yl)methyl; morpholin-4-ylmethyl; or (2-oxo-piperazin-4-yl)methyl. More preferably, R⁴ is piperazin-1-ylmethyl; 4-methylpiperazin-1-yl; 4-ethylpiperazin-1-yl; morpholin-4-yl; (4-methylpiperazin-1-yl)methyl; (4-ethylpiperazin-1-yl)methyl; or morpholin-4-ylmethyl; More preferably, R⁴ is 4-methylpiperazin-1-yl; 4-ethylpiperazin-1-yl; morpholin-4-yl; (4-methylpiperazin-1-yl)methyl; morpholin-4-ylmethyl; or (2-oxo-piperazin-4-yl)methyl. Even more preferably, R⁴ is 4-methylpiperazin-1-yl; 4-ethylpiperazin-1-yl; morpholin-4-yl; (4-methylpiperazin-1-yl)methyl; or morpholin-4-ylmethyl.

Preferably, R³ is H.

Preferably, R², R^{2a} are independently selected from the group consisting of H; F; and Cl.

In the method of the present invention compounds of the formula (I) in which some or all of the above-mentioned groups have the preferred or more preferred meanings are also an object of the present invention.

Preferred specific compounds of formula (I) in the method of the present invention are selected from the group consisting of
1-((4-{2-[2-(3,5-dimethoxyphenyl) ethyl]-5H-pyrrolo [2, 3-b] pyrazin-6-yl} phenyl)-4-methylpiperazine;
1-((4-{2-[(E)-2-(3,5-dimethoxyphenyl) ethenyl] 5H-pyrrolo [2,3-b] pyrazin-6-yl} phenyl)4-methylpiperazine;
1-((4-{2-[2-(3,5-dimethoxyphenyl) ethyl]-5H-pyrrolo [2,3-b] pyrazine-6-yl} phenyl) piperazine;
1-((4-{2-[(E)-2-(3,5-dimethoxyphenyl) ethenyl]-5H-pyrrolo [2,3-b] pyrazin-6-yl} phenyl) piperazine;
N-[(3,5-dimethoxyphenyl) methyl]-6-[4-(4-methylpiperazin-1-yl) phenyl]-5H-pyrrolo [2,3-b] pyrazin-2-amine;
N-[(2, 6-dichloro-3,5-dimethoxyphenyl) methyl]-6-[4-(4-methylpiperazin-1-yl) phenyl]-5H-pyrrolo [2,3-b] pyrazin-2-amine;
N-[(3,5-dimethoxyphenyl) methyl]-6-[4-(piperazin-1-yl) phenyl]-5H-pyrrolo [2,3-b] pyrazin-2-amine; and
1-((4-{5-[(2,6-dichloro-3,5-dimethoxyphenyl) methoxy]-1H-pyrrolo [2,3-b] pyridin-2-yl} phenyl)-4-methylpiperazine.

Further preferred specific compounds of formula (I) in the method of the present invention are selected from the group consisting of
N-[(2,6-dichloro-3,5-dimethoxyphenyl)methyl]-6-{5-[(4-methylpiperazin-1-yl)methyl] pyridin-2-yl}-5H-pyrrolo[2,3-b]pyrazin-2-amine;
N-[(2,6-dichloro-3,5-dimethoxyphenyl)methyl]-6-{5-[(4-ethylpiperazin-1-yl)methyl]pyridin-2-yl}-5H-pyrrolo[2,3-b]pyrazin-2-amine;
N-[(2,6-dichloro-3,5-dimethoxyphenyl)methyl]-6-[5-(morpholin-4-ylmethyl)pyridin-2-yl]-5H-pyrrolo[2,3-b]pyrazin-2-amine; and
N-[(2,6-dichloro-3,5-dimethoxyphenyl)methyl]-6-[5-(piperazin-1-ylmethyl)pyridin-2-yl]-5H-pyrrolo[2,3-b]pyrazin-2-amine.

Further preferred specific compounds of formula (I) in the method of the present invention are selected from the group consisting of
1-((4-{2-[2-(2,6-dichloro-3,5-dimethoxyphenyl)ethyl]-5H-pyrrolo[2,3-b]pyrazin-6-yl}phenyl)-4-methylpiperazine;
1-((4-{5-[2-(3,5-Dimethoxyphenyl)ethyl]-1H-pyrrolo[2,3-b] pyridin-2-yl}phenyl)-4-methylpiperazine; and
1-((4-{2-[2-(2,6-difluoro-3,5-dimethoxyphenyl)ethyl]-5H-pyrrolo[2,3-b]pyrazin-6-yl}phenyl)-4-methylpiperazine.

In a preferred method of the present invention, wherein Y¹ is I, the method comprises before step (a₀) a step
(a₋₁) iodination of a compound of formula (II) 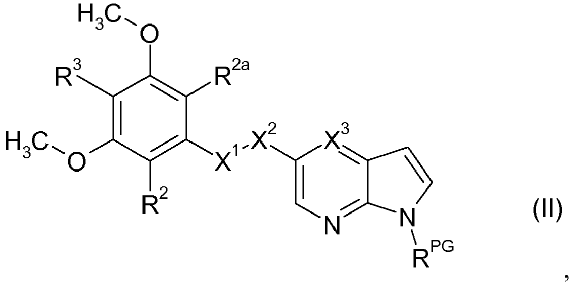 wherein R², R^{2a}, R³, X¹, X², X³ have the meaning as indicated above and R^{PG} is PG, resulting in a compound of (Ia) 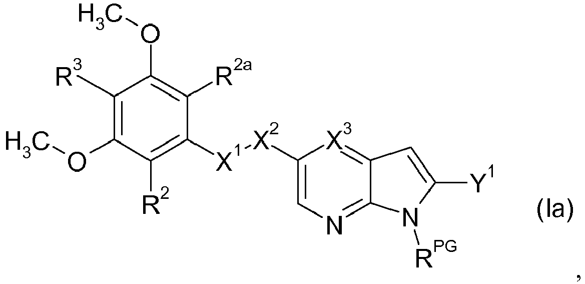 wherein R², R^{2a}, R³, X¹, X², X³ have the meaning as indicated above, Y¹ is I and R^{PG} is PG.

Preferably, iodination is carried out using iodine.

Preferably, between step (a₋₁) and (a₀) is a step of deprotecting a compound of formula (Ia), wherein R², R^{2a}, R³, X¹, X², X³ have the meaning as indicated above, Y¹ is I and R^{PG} is PG, resulting in a compound of formula (Ia), wherein R², R^{2a}, R³, X¹, X², X³ have the meaning as indicated above, Y¹ is I and R^{PG} is H.

Preferably, before step (a₀) a compound of formula (Ia), wherein R², R^{2a} are H; R³, X¹, X², X³ have the meaning as indicated above; Y¹ is I and R^{PG} is H or PG, is chlorinated resulting in a compound of formula (Ia), wherein R², R^{2a} are Cl; R³, X¹, X², X³ have the meaning as indicated above; Y¹ is I and R^{PG} is H or PG.

A further aspect of the present invention is a compound of formula (Ia) as indicated above, preferably wherein Y¹ is I. Preferably, R³ is H. Preferably, R², R^{2a} are independently selected from the group consisting of H; F; and Cl. Preferably, R², R^{2a} are both H; F or Cl. Preferably, R^{PG} is H. Also preferably, R^{PG} is PG, especially phenylsulfonyl or 4-phenylsulfonyl. Such compounds are useful as intermediates for the preparation of compounds of formula (I).

Preferred compounds of formula (Ia) are selected from the group consisting of
5-((Benzenesulfonyl)-2-[2-(3,5-dimethoxyphenyl)ethyl]-6-iodo-5H-pyrrolo[2,3-b]pyrazine;
2-[2-(3,5-Dimethoxyphenyl)ethyl]-6-iodo-5-(4- methylbenzenesulfonyl)-5H-pyrrolo[2,3-b]pyrazine;
2-[2-(3,5-Dimethoxyphenyl)ethyl]-6-iodo-5H-pyrrolo[2,3-b]pyrazine;
2-[2-(2,6-Dichloro-3,5-dimethoxyphenyl)ethyl]-6-iodo-5-(4-methylbenzenesulfonyl)-5H-pyrrolo[2,3-b]pyrazine;
1-((Benzenesulfonyl)-5-[2-(3,5-dimethoxyphenyl)ethyl]-2-iodo-1H-pyrrolo[2,3-b]pyridine;
1-((Benzenesulfonyl)-5-[2-(2,6-dichloro-3,5-dimethoxyphenyl)ethyl]-2-iodo-1H-pyrrolo[2,3-b]pyridine; and
5-((benzenesulfonyl)-2-[2-(2,6-difluoro-3,5-dimethoxyphenyl)ethyl]-6-iodo-5H-pyrrolo[2,3-b]pyrazine.

Where tautomerism, like e.g. keto-enol tautomerism, of compounds of formula (I), (Ia), (Ib), (Ic), or (II) may occur, the individual forms, like e.g. the keto and enol form, are comprised separately and together as mixtures in any ratio. Same applies for stereoisomers, like e.g. enantiomers, cis/trans isomers, conformers and the like.

Especially, when enantiomeric or diastereomeric forms are given in a compound according to formula (I), (Ia), (Ib), (Ic), or (II) each pure form separately and any mixture of at least two of the pure forms in any ratio is comprised by formula (I), (Ia), (Ib), (Ic), or (II) and is a subject of the present invention.

Isotopic labeled compounds of formula (I), (Ia), (Ib), (Ic), or (II) are also within the scope of the present invention. Methods for isotope labeling are known in the art. Preferred isotopes are those of the elements H, C, N, O and S. Solvates of compounds of formula (I), (Ia), (Ib), (Ic), or (II) are also within the scope of the present invention.

If desired, isomers can be separated by methods well known in the art, e.g. by liquid chromatography. Same applies for enantiomers by using e.g. chiral stationary phases. Additionally, enantiomers may be isolated by converting them into diastereomers, i.e. coupling with an enantiomerically pure auxiliary compound, subsequent separation of the resulting diastereomers and cleavage of the auxiliary residue. Alternatively, any enantiomer of a compound of formula (I), (Ia), (Ib), (Ic), or (II) may be obtained from stereoselective synthesis using optically pure starting materials, reagents and/or catalysts.

In case the compounds according to formula (I), (Ia), (Ib), (Ic), or (II) contain one or more acidic or basic groups, the invention also comprises their corresponding pharmaceutically or toxicologically acceptable salts, in particular their pharmaceutically utilizable salts. Thus, the compounds of the formula (I), (Ia), (Ib), (Ic), or (II) which contain acidic groups can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I), (Ia), (Ib), (Ic), or (II) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I), (Ia), (Ib), (Ic), or (II) simultaneously contain acidic and basic groups in the molecule, the invention also includes, in addition to the salt forms mentioned, inner salts or betaines (zwitterions). The respective salts according to the formula (I), (Ia), (Ib), (Ic), or (II) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I), (Ia), (Ib), (Ic), or (II) which, owing to low physiological compatibility, are not directly suitable for use in pharmaceuticals but which can be used, for example, as intermediates for chemical reactions or for the preparation of pharmaceutically acceptable salts.

Starting materials for the synthesis of preferred embodiments of the invention may be purchased from commercially available sources such as Array, Sigma Aldrich, Acros, Fisher, Fluka, ABCR or can be synthesized using known methods by one skilled in the art.

In general, several methods are applicable to prepare compounds of the present invention. In some cases various strategies can be combined. Sequential or convergent routes may be used. Exemplary synthetic routes are described below. It is clear to a practitioner in the art that functional groups may be protected in a preparation step followed by deprotection. Accordingly compounds described herein in their protected form are also within the scope of the present invention.

### Examples

### Abbreviations

- aq: Aqueous
- Brine: Saturated solution of sodium chloride in water
- DIPEA: N,N-Diisopropylethylamine
- DMF: N,N-Dimethylformamide
- EtOAc: Ethyl acetate
- EtOH: Ethanol
- EtO₂: Diethyl ether
- FCC: Flash column chromatography
- g: Gram (s)
- h: Hour (s)
- HPLC: High-performance liquid chromatography
- IPC: In process check
- LDA: Lithium diisopropylamide
- MeCN: Acetonitrile
- MeOH: Methanol
- Min (s): Minute (s)
- mg: Milligram (s)
- ml: Millilitre (s)
- mm: Millimetre (s)
- mM: Millimolar
- NaHCO₃: Sodium hydrogen carbonate
- NaOH: Sodium hydroxide
- Na₂S₂O₃: Sodium thiosulfate
- NH₃: Ammonia
- NH₄Cl: Ammonium chloride
- NMR: Nuclear Magnetic Resonance
- RT: Room temperature
- SCX: Cation-exchange columns with propylsulfonic acid-tailed silica
- THF: Tetrahydrofuran
- TBME: Methyl tert-butyl ether
- t_{R}: Time of retention
- µl: Microlitre (s)
- µm: Micrometre (s)

**Analytical LCMS conditions are as follows:**
**System 1: ACIDIC IPC 2 min METHOD:** Linear gradient 5-100 % solvent B in 1.5 mins + 0.1 mins 100 % solvent B, flow rate 1ml/min. Column Supelco Ascentis Express Part No. 53802-U (30 X 2.1 mm, 1.7 µm). Solvent A = 0.1 % Formic acid in water, Solvent B = 0.1 % Formic acid in Acetonitrile.
**System 2: BASIC IPC 7 min METHOD:** Linear gradient 5-100 % solvent B in 5.5 mins + 0.4 mins 100 % solvent B, flow rate 0.5ml/min. Column Phenomenex Gemini-NX C18 Part No. 00D-4453-B0 (100 X 2.0 mm, 3 µm). Solvent A = 2 mM ammonium bicarbonate, buffered to pH 10, Solvent B = Acetonitrile.
**System 3: ACIDIC FINAL 7 min METHOD:** Linear gradient 5-100 % solvent B in 5.3 mins + 0.5 mins 100 % solvent B, flow rate 0.6ml/min. Column Phenomenex Kinetix-XB C18 Part No. 00D-4498-AN (100 X 2.1 mm, 1.7 µm). Solvent A = 0.1 % Formic acid in water, Solvent B = 0.1 % Formic acid in Acetonitrile.

### Synthetic Routes

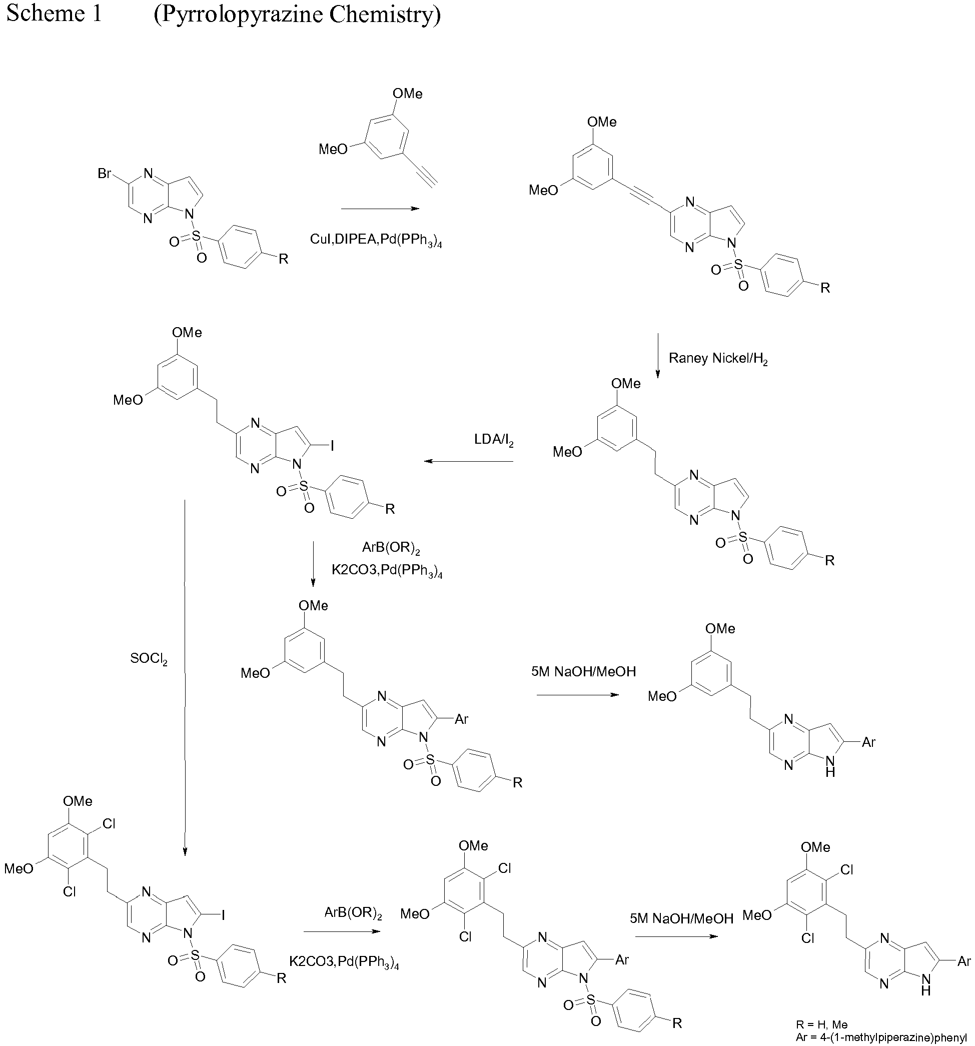

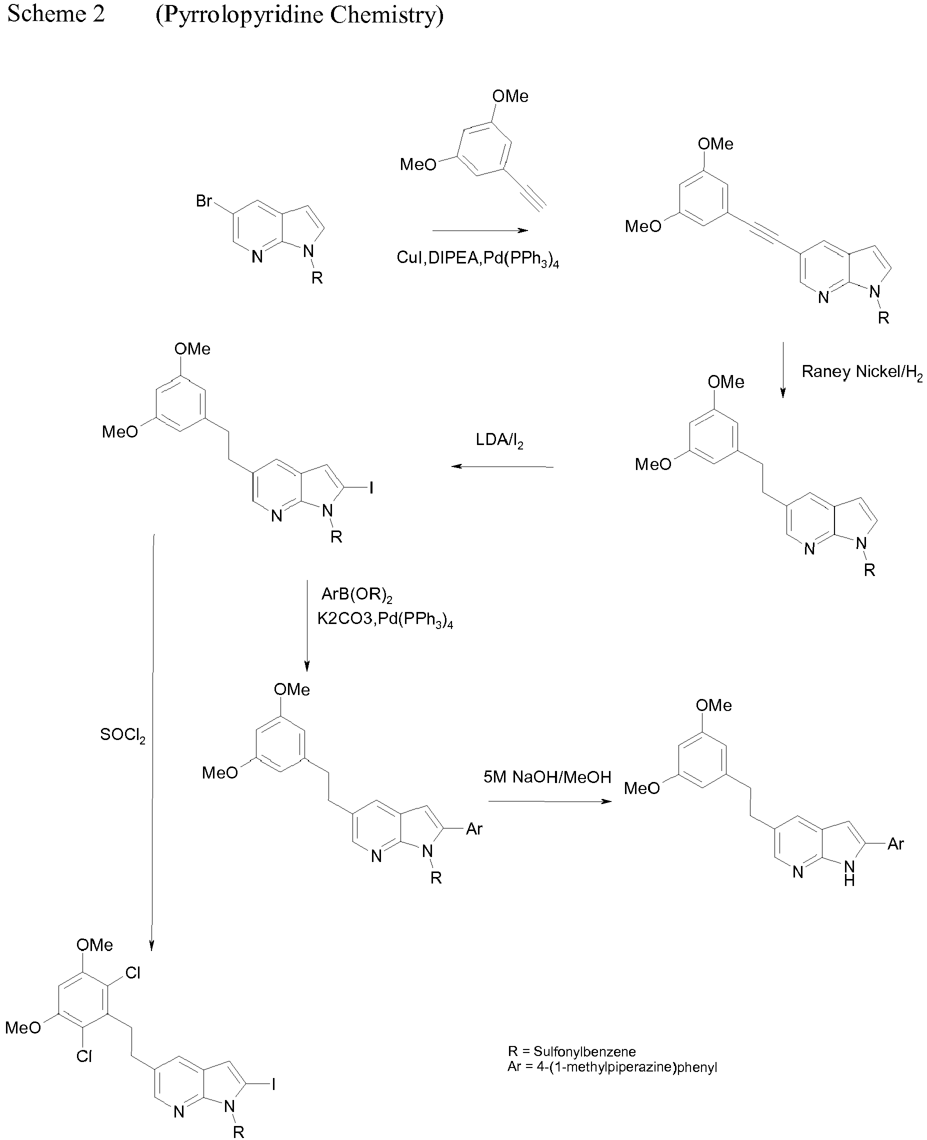

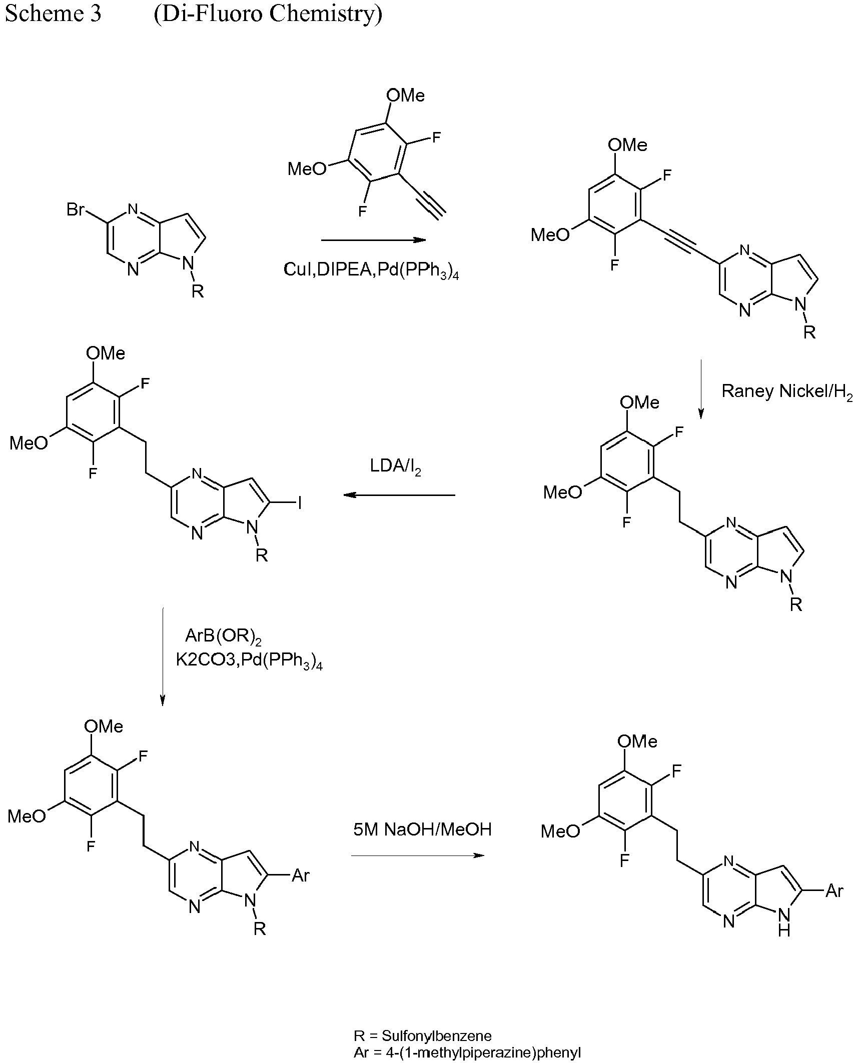

### Experimental

### Pyrrolopyrazine Chemistry

### 5-(Benzenesulfonyl)-2-[2-(3,5-dimethoxyphenyl)ethynyl]-5H-pyrrolo[2,3-b]pyrazine

5-(Benzenesulfonyl)-2-bromo-5H-pyrrolo[2,3-b]pyrazine (3.50 g, 10.35 mmol), copper(1+) iodide (197.1 mg, 1.03 mmol) and palladium tetrakis triphenylphospine (597.7 mg, 0.52 mmol) were dissolved in MeCN (40 ml). Reaction mixture was degassed with nitrogen for 5 mins. 1-ethynyl-3,5-dimethoxybenzene (2.52 g, 15.52 mmol) and DIPEA (8.96 ml, 51.75 mmol) were added and reaction heated to 80°C, under a nitrogen atmosphere, for 2 h. Reaction mixture was allowed to cool to RT, then to 0°C and left to stand overnight. The beige precipitate formed after this time was filtered off, dissolved in EtOAc, washed with water and concentrated. Crude material was re-crystallised from EtOAc to give the title compound as a yellow solid (3.16 g, 73%).
¹H NMR (500 MHz, Chloroform-*d*) δ 8.55 (s, 1H), 8.24 - 8.13 (m, 2H), 8.04 (d, *J* = 3.7 Hz, 1H), 7.66 - 7.60 (m, 1H), 7.52 (t, *J* = 7.9 Hz, 2H), 6.82 (s, 1H), 6.77 (d, *J* = 2.2 Hz, 2H), 6.51 (t, *J* = 2.2 Hz, 1H), 3.80 (s, 6H). M/Z: 419.09, M+1: 419.95, t_{R}= 1.59 min (System 1).

### 2-[2-(3,5-Dimethoxyphenyl)ethynyl]-5-(4-methylbenzenesulfonyl)-5H-pyrrolo[2,3-b]pyrazine

The title compound was prepared in analogy to the procedure described above using 2-bromo-5-(4-methylbenzenesulfonyl)-5H-pyrrolo[2,3-b]pyrazine (6.20 g, 17.60 mmol), copper(1+) iodide (335.3 mg, 1.76 mmol), palladium tetrakis triphenylphospine (1.02 g, 0.88 mmol), 1-ethynyl-3,5-dimethoxybenzene (4.28 g, 26.40 mmol), DIPEA (15.23 ml, 88.00 mmol) and MeCN (120 ml) to afford the title compound (6.70 g, 88%). In this case purification was done by trituration with Et₂O.
¹H NMR (500 MHz, Chloroform-*d*) δ 8.55 (s, 1H), 8.08 - 8.01 (m, 3H), 7.31 (d, *J* = 8.1 Hz, 2H), 6.84 - 6.75 (m, 3H), 6.51 (t, *J* = 2.3 Hz, 1H), 3.80 (s, 6H), 2.40 (s, 3H). M/Z: 433.11, M+1: 433.95, t_{R}= 1.59 min (System 1).

### 5-(Benzenesulfonyl)-2-[2-(3,5-dimethoxyphenyl)ethyl]-5H-pyrrolo[2,3-b]pyrazine

5-(Benzenesulfonyl)-2-[2-(3,5-dimethoxyphenyl)ethynyl]-5H-pyrrolo[2,3-b]pyrazine (1.52 g, 3.62 mmol) was dissolved in 1:1 EtOH:DMF (12 ml) and Raney Nickel (2.4 µl, 0.36 mmol) (10 mol % slurry in water) added. Reaction was placed under an atmosphere of hydrogen and allowed to stir at RT for 16 h. Reaction was filtered through celite and washed with copious amounts of MeOH. Filtrate was concentrated and DMF washed out by partitioning residue between water and EtOAc. Organics were combined and concentrated. Crude material was purified by FCC on Biotage (0 - 100% EtOAc / Heptane) to give the title compound as a yellow / orange solid (1.26 g, 82%).
¹H NMR (500 MHz, Chloroform-*d*) δ 8.20 - 8.14 (m, 3H), 7.97 (d, *J* = 4.1 Hz, 1H), 7.63 - 7.58 (m, 1H), 7.50 (t, *J* = 7.9 Hz, 2H), 6.80 (d, *J* = 4.1 Hz, 1H), 6.33 (d, *J* = 2.2 Hz, 2H), 6.30 (t, *J* = 2.2 Hz, 1H), 3.73 (s, 6H), 3.17 (dd, *J* = 9.5, 6.6 Hz, 2H), 3.00 (dd, *J* = 9.5, 6.6 Hz, 2H). M/Z: 423.13, M+1: 423.95, t_{R}= 1.48 min (System 1).

### 2-[2-(3,5-Dimethoxyphenyl)ethyl]-5-(4-methylbenzenesulfonyl)-5H-pyrrolo[2,3-b]pyrazine

The title compound was prepared in analogy to the procedure described above using 2-[2-(3,5-dimethoxyphenyl)ethynyl]-5-(4-methylbenzenesulfonyl)-5H-pyrrolo[2,3-b]pyrazine (3.54 g, 8.17 mmol), Raney Nickel (5.4 µl, 0.82 mmol) (10 mol % slurry in water) and EtOH (100 ml) to afford the title compound as a yellow / orange solid (2.89 g, 81%). In this case purification was done by FCC on Biotage (0 - 50% EtOAc / Heptane).
¹H NMR (500 MHz, Chloroform-*d*) δ 8.17 (s, 1H), 8.04 (d, *J* = 8.4 Hz, 2H), 7.97 (d, *J* = 4.1 Hz, 1H), 7.29 (d, *J* = 8.1 Hz, 2H), 6.79 (d, *J* = 4.1 Hz, 1H), 6.34 (d, *J* = 2.2 Hz, 2H), 6.30 (t, *J* = 2.2 Hz, 1H), 3.73 (s, 6H), 3.17 (dd, *J* = 9.5, 6.6 Hz, 2H), 3.00 (dd, *J* = 9.5, 6.6 Hz, 2H), 2.39 (s, 3H). M/Z: 437.14, M+1: 437.95, t_{R}= 1.53 min (System 1).

### 5-(Benzenesulfonyl)-2-[2-(3,5-dimethoxyphenyl)ethyl]-6-iodo-5H-pyrrolo[2,3-b]pyrazine

THF (4 ml) was added to a cooled (-78°C) solution of LDA (2M in THF, 0.43 ml) under nitrogen. A solution of 5-(benzenesulfonyl)-2-[2-(3,5-dimethoxyphenyl)ethyl]-5H-pyrrolo[2,3-b]pyrazine (300.0 mg, 0.71 mmol) dissolved in THF (2 ml) was added drop wise over 10 mins and the reaction left to stir at -78°C for 2 h. A solution of iodine (233.7 mg, 0.92 mmol) in THF (3 ml) was added drop wise at -78°C and the reaction allowed to stir for 1 h. Reaction mixture was quenched at -78°C with saturated aq. NH₄Cl solution and allowed to warm to RT. Brine was added, then organics extracted with EtOAc, dried, filtered and concentrated. Crude material was purified by FCC on Biotage (15 - 50% EtOAc / Heptane) to give the title compound (310.0 mg, 80%).
¹H NMR (500 MHz, Chloroform-*d*) δ 8.18 (dd, *J* = 8.5, 1.2 Hz, 2H), 8.11 (s, 1H), 7.65 - 7.58 (m, 1H), 7.55 - 7.48 (m, 2H), 7.20 (s, 1H), 6.34 - 6.30 (m, 3H), 3.74 (s, 6H), 3.17 (dd, *J* = 9.3, 6.6 Hz, 2H), 3.00 (dd, *J* = 9.3, 6.6 Hz, 2H). M/Z: 549.02, M+1: 549.80, t_{R}= 1.54 min (System 1).

### 2-[2-(3,5-Dimethoxyphenyl)ethyl]-6-iodo-5-(4- methylbenzenesulfonyl)-5H-pyrrolo[2,3-b]pyrazine

The title compound was prepared in analogy to the procedure described above using 2-[2-(3,5-dimethoxyphenyl)ethyl]-5-(4-methylbenzenesulfonyl)-5H-pyrrolo[2,3-b]pyrazine (750.0 mg, 1.71 mmol), LDA (2M in THF, 1.03 ml), iodine (565.6 mg, 2.23 mmol) and THF (16 ml) to afford the title compound (801.0 mg, 83%). In this case purification was done by FCC on Biotage (20 - 50% EtOAc / Heptane).
¹H NMR (250 MHz, Chloroform-*d*) δ 8.23 - 7.90 (m, 3H), 7.29 (d, *J* = 8.1 Hz, 2H), 7.18 (s, 1H), 6.33 (d, *J* = 2.1 Hz, 2H), 6.31 (d, *J* = 2.1 Hz, 1H), 3.73 (d, *J* = 2.1 Hz, 6H), 3.26 - 3.10 (m, 2H), 2.99 (dd, *J* = 9.3, 6.1 Hz, 2H), 2.39 (s, 3H). M/Z: 563.04, M+1: 563.80, t_{R}= 1.61 min (System 1).

### 2-[2-(3,5-Dimethoxyphenyl)ethyl]-6-iodo-5H-pyrrolo[2,3-b]pyrazine

5-(Benzenesulfonyl)-2-[2-(3,5-dimethoxyphenyl)ethyl]-6-iodo-5H-pyrrolo[2,3-b]pyrazine or 2-[2-(3,5-dimethoxyphenyl)ethyl]-6-iodo-5-(4-methylbenzenesulfonyl)-5H-pyrrolo[2,3-b]pyrazine (1 eq) was dissolved in MeOH (2 - 5 ml) and NaOH (5M in water, 5 eq) added. Reaction was stirred at 60°C for 1 h. Reaction mixture was cooled to RT and concentrated under vacuum to give the title compound as a 1:1 mixture of product and des-iodo product. No further purification was done.
M/Z: 409.03, M+1: 409.90, t_{R}= 1.31 min (System 1).

### 1-(4-{2-[2-(3,5-Dimethoxyphenyl)ethyl]-5H-pyrrolo[2,3-b]pyrazin-6-yl}phenyl)-4-methylpiperazine

2-[2-(3,5-Dimethoxyphenyl)ethyl]-6-iodo-5H-pyrrolo[2,3-b]pyrazine (120.0 mg, 0.29 mmol), 1-methyl-4-[4-(tetramethyl-1,3,2-dioxaborolan-2-yl)phenyl]piperazine (97.5 mg, 0.32 mmol) and potassium carbonate hydrate (4:2:3) (290.7 mg, 0.88 mmol) were dissolved in 1:1 water:dioxane and reaction mixture degassed with nitrogen for 5 mins. Palladium tetrakis triphenylphospine (33.9 mg, 0.03 mmol) was added and reaction vessel sealed and heated to 100°C for 16 h. Reaction mixture was cooled to RT and concentrated. Residue was diluted with water and extracted into EtOAc (x 3). Organics were combined, concentrated to 5 ml and loaded directly onto an SCX column. MeOH / NH₃ fractions were collected, concentrated to dryness and triturated with hot MeOH, followed by EtOAc, to give the title compound as a brown solid (52.0 mg, 39%).
¹H NMR (250 MHz, DMSO-*d*₆) δ 12.11 (s, 1H), 7.99 (s, 1H), 7.84 (d, *J*= 8.7 Hz, 2H), 7.04 (d, *J* = 8.7 Hz, 2H), 6.87 (s, 1H), 6.40 (d, *J* = 1.9 Hz, 2H), 6.30 (s, 1H), 3.69 (s, 6H), 3.27 - 3.23 (m, 4H), 3.07 (d, *J* = 8.3 Hz, 2H), 2.98 (d, *J* = 8.3 Hz, 2H), 2.47 - 2.42 (m, 4H), 2.23 (s, 3H). M/Z: 457.25, M+1: 458.20, t_{R}= 4.28 min (System 2).

### 2-[2-(2,6-Dichloro-3,5-dimethoxyphenyl)ethyl]-6-iodo-5-(4-methylbenzenesulfonyl)-5H-pyrrolo [2,3-b] pyrazine

2-[2-(3,5-dimethoxyphenyl)ethyl]-6-iodo-5-(4-methylbenzenesulfonyl)-5H-pyrrolo[2,3-b]pyrazine (200.0 mg, 0.35 mmol) was suspended in Et₂O (10 ml) and cooled to 0°C. Sulfuryl dichloride (115.1 µl, 1.42 mmol) was added and reaction was warmed to RT with stirring for 90 mins. Reaction mixture was filtered and washed with TBME to give the title compound as a yellow / green solid (203.0 mg, 90%).
¹H NMR (250 MHz, DMSO-*d*₆) δ 8.20 (s, 1H), 7.90 (d, *J*= 8.3 Hz, 2H), 7.45 (d, *J*= 8.3 Hz, 2H), 7.43 (s, 1H), 6.85 (s, 1H), 3.91 (s, 6H), 3.34 - 3.21 (m, 2H), 3.08 - 2.98 (m, 2H), 2.36 (s, 3H). M/Z: 630.96, M+1: 631.70, t_{R}= 1.71 min (System 1).

### 1-(4-{2-[2-(2,6-dichloro-3,5-dimethoxyphenyl)ethyl]-5H-pyrrolo[2,3-b]pyrazin-6-yl}phenyl)-4-methylpiperazine

2-[2-(2,6-Dichloro-3,5-dimethoxyphenyl)ethyl]-6-iodo-5-(4-methylbenzenesulfonyl)-5H-pyrrolo[2,3-b]pyrazine (33.0 mg, 0.05 mmol), 1-methyl-4-[4-(tetramethyl-1,3,2-dioxaborolan-2-yl)phenyl]piperazine (18.1 mg, 0.06 mmol) and potassium carbonate hydrate (4:2:3) (51.7 mg, 0.16 mmol) were dissolved in 1:1 water:dioxane (1 ml) and reaction mixture degassed with nitrogen for 5 mins. Palladium tetrakis triphenylphospine (6.0 mg, 0.01 mmol) was added and reaction vessel sealed and heated to 100°C for 16 h. NaOH (5M in water, 1 ml) was added and reaction heated to 60°C for 1 h. Reaction mixture was cooled to RT and concentrated. Residue was diluted with water and extracted into EtOAc (x 3). Organics were combined, concentrated to 5 ml and loaded directly onto an SCX column. MeOH / NH₃ fractions were collected, concentrated to dryness and triturated with hot MeOH to give the title compound as a pale yellow solid (10.0 mg, 36%).
¹H NMR (500 MHz, DMSO-*d*₆) δ 12.15 (s, 1H), 7.98 (d, *J*= 4.6 Hz, 1H), 7.84 (d, *J*= 8.8 Hz, 2H), 7.04 (d, *J*= 8.8 Hz, 2H), 6.90 (d, *J*= 1.4 Hz, 1H), 6.84 (s, 1H), 3.91 (s, 6H), 3.27 - 3.23 (m, 4H), 3.19 - 3.02 (m, 2H), 3.01 - 2.93 (m, 2H), 2.48 - 2.43 (m, 4H), 2.23 (s, 3H). M/Z: 525.17, M+1: 526.10, t_{R}= 2.31 min (System 3).

### Pyrrolopyridine Chemistry

### 1-(Benzenesulfonyl)-5-[2-(3,5-dimethoxyphenyl)ethynyl]-1H-pyrrolo[2,3-b]pyridine

1-(Benzenesulfonyl)-5-bromo-1H-pyrrolo[2,3-b]pyridine (639.0 mg, 1.90 mmol), 1-ethynyl-3,5-dimethoxybenzene (461.0 mg, 2.84 mmol), copper(1+) iodide (36.1 mg, 0.19 mmol) and DIPEA (1.62 ml, 9.48 mmol) were dissolved in MeCN (10 ml). Reaction mixture was degassed with nitrogen for 5 mins. Palladium tetrakis triphenylphospine (109.5 mg, 0.09 mmol) was added and reaction heated to 80°C in a sealed tube for 90 mins. Reaction mixture was cooled to RT, partitioned between saturated aq. NaHCO₃ solution and EtOAc and organics separated. Organics were washed with brine, dried, filtered and concentrated. Crude material was purified by FCC on Biotage (0 - 50% EtOAc / Heptane) to give the title compound as a yellow solid (867.0 mg, 100%).
¹H NMR (500 MHz, Chloroform-*d*) δ 8.59 (d, *J* = 1.9 Hz, 1H), 8.27 - 8.12 (m, 2H), 8.00 (d, *J* = 1.9 Hz, 1H), 7.78 (d, *J* = 4.0 Hz, 1H), 7.61 (d, *J* = 7.5 Hz, 1H), 7.52 (t, *J* = 7.8 Hz, 2H), 6.71 (d, *J* = 2.3 Hz, 2H), 6.62 (d, *J* = 4.0 Hz, 1H), 6.50 (t, *J* = 2.3 Hz, 1H), 3.83 (s, 6H). M/Z: 418.10, M+1: 418.90, t_{R}= 1.63 min (System 1).

### 1-(Benzenesulfonyl)-5-[2-(3,5-dimethoxyphenyl)ethyl]-1H-pyrrolo[2,3-b]pyridine

1-(Benzenesulfonyl)-5-[2-(3,5-dimethoxyphenyl)ethynyl]-1H-pyrrolo[2,3-b]pyridine (1.90 g, 4.54 mmol) was dissolved in 1:1 EtOH:DMF (20 ml) and Raney Nickel (3.0 µl, 0.45 mmol) (10 mol % slurry in water) added. Reaction was placed under an atmosphere of hydrogen and allowed to stir at RT for 40 h. A further Raney Nickel (3.0 µl, 0.45 mmol) (10 mol % slurry in water) was added and reaction stirred under an atmosphere of hydrogen for 24 h. Reaction was filtered through celite and washed with copious amounts of MeOH. Filtrate was concentrated and DMF washed out by partitioning residue between water and EtOAc. Organics were combined and concentrated to give the title compound as a green solid (932.0 mg, 49%).
¹H NMR (250 MHz, Chloroform-*d*) δ 8.25 (d, *J*= 2.0 Hz, 1H), 8.17 (dd, *J*= 7.0, 1.6 Hz, 2H), 7.68 (d, *J* = 4.0 Hz, 1H), 7.63 - 7.42 (m, 4H), 6.52 (d, *J* = 4.0 Hz, 1H), 6.34 - 6.23 (m, 3H), 3.72 (s, 6H), 3.07 - 2.92 (m, 2H), 2.85 (dd, *J*= 9.0, 6.1 Hz, 2H). M/Z: 422.13, M+1: 422.95, t_{R}= 1.56 min (System 1).

### 1-(Benzenesulfonyl)-5-[2-(3,5-dimethoxyphenyl)ethyl]-2-iodo-1H-pyrrolo[2,3-b]pyridine

1-(Benzenesulfonyl)-5-[2-(3,5-dimethoxyphenyl)ethyl]-1H-pyrrolo[2,3-b]pyridine (628.0 mg, 1.49 mmol) was dissolved in THF (20 ml) and cooled to -78°C. LDA (2M in THF, 0.89 ml) was added over 1 min and the reaction allowed to stir at -78°C for 2 h. At this point a solution of iodine (490.4 mg, 1.93 mmol) in THF (10 ml) was added at -78°C and the reaction stirred for 10 mins. Reaction mixture was quenched at -78°C with saturated aq. NaHCO₃ solution and allowed to warm to RT. Organics were extracted into EtOAc and combined organics were washed with 10% aq. Na₂S₂O₃ solution, dried, filtered and concentrated. Crude material was purified by FCC on Biotage (0 - 50% EtOAc / Heptane) to give the title compound (295.0 mg, 36%).
¹H NMR (500 MHz, Chloroform-*d*) δ 8.22 - 8.18 (m, 3H), 7.57 (td, *J*= 7.2, 1.1 Hz, 1H), 7.52 - 7.44 (m, 3H), 6.91 (s, 1H), 6.30 (t, *J* = 2.2 Hz, 1H), 6.27 (d, *J* = 2.2 Hz, 2H), 3.73 (s, 6H), 2.97 (dd, *J* = 9.0, 6.6 Hz, 2H), 2.84 (dd, *J* = 9.0, 6.6 Hz, 2H). M/Z: 548.03, M+1: 548.80, t_{R}= 1.59 min (System 1).

### 1-(4-{5-[2-(3,5-Dimethoxyphenyl)ethyl]-1H-pyrrolo[2,3-b]pyridin-2-yl}phenyl)-4-methylpiperazine

1-(Benzenesulfonyl)-5-[2-(3,5-dimethoxyphenyl)ethyl]-2-iodo-1H-pyrrolo[2,3-b]pyridine (115.0 mg, 0.21 mmol), 1-methyl-4-[4-(tetramethyl-1,3,2-dioxaborolan-2-yl)phenyl]piperazine (72.9 mg, 0.24 mmol) and potassium carbonate hydrate (4:2:3) (207.8 mg, 0.63 mmol) were dissolved in 1:1 water:dioxane (3 ml) and reaction mixture degassed with nitrogen for 5 mins. Palladium tetrakis triphenylphospine (24.2 mg, 0.02 mmol) was added and reaction vessel sealed and heated to 100°C for 3 h. Reaction mixture was cooled to RT and concentrated. Residue was diluted with water and extracted into EtOAc (x 3). Organics were combined, concentrated to 5 ml and loaded directly onto an SCX column. MeOH / NH₃ fractions were collected and concentrated to dryness. Resulting material was dissolved in MeOH (2 ml) and NaOH (5M in water, 1 ml) was added. Reaction was heated to 50°C for 2 h. Reaction mixture was cooled to RT, extracted into EtOAc (x 3) and washed with water. Crude material was purified by SCX column, followed by preparative basic HPLC to give the title compound as a yellow solid (15.5 mg, 16%).
¹H NMR (250 MHz, DMSO-*d*₆) δ 11.77 (s, 1H), 7.99 (s, 1H), 7.76 (d, *J* = 8.8 Hz, 2H), 7.69 (s, 1H), 7.00 (d, *J* = 8.8 Hz, 2H), 6.64 (s, 1H), 6.41 (d, *J* = 2.0 Hz, 2H), 6.30 (s, 1H), 3.70 (s, 6H), 3.23 - 3.19 (m, 4H), 2.91 - 2.87 (m, 4H), 2.47 - 2.42 (m, 4H), 2.23 (s, 3H). M/Z: 456.25, M+1: 457.30, t_{R}= 4.84 min (System 2).

### 1-(Benzenesulfonyl)-5-[2-(2,6-dichloro-3,5-dimethoxyphenyl)ethyl]-2-iodo-1H-pyrrolo [2,3-b] pyridine

5-[2-(3,5-Dimethoxyphenyl)ethyl]-2-iodo-1-(4-methylbenzenesulfonyl)-1H-pyrrolo[2,3-b]pyridine (236.0 mg, 0.43 mmol) was suspended in Et₂O (10 ml) and cooled to 0°C. Sulfuryl dichloride (340.2 µl, 4.20 mmol) was added and reaction stirred at 0°C for 20 mins. Reaction mixture was filtered and the resultant solid triturated in EtOAc to give the title compound as a yellow / green solid (65.7 mg, 10%).
¹H NMR (250 MHz, Chloroform-*d*) δ 8.29 (d, *J* = 2.1 Hz, 1H), 8.24 - 8.16 (m, 2H), 7.64 - 7.47 (m, 4H), 6.94 (s, 1H), 6.49 (s, 1H), 3.92 (s, 6H), 3.22 (dd, *J* = 10.0, 6.5 Hz, 2H), 2.90 (dd, *J*= 10.0, 6.5 Hz, 2H). M/Z: 615.95, M+1: 616.70, t_{R}= 1.72 min (System 1).

### Di-Fluoro Chemistry

### 3-Ethynyl-2,4-difluoro-1,5-dimethoxybenzene

Selectfluor (24.57 g, 69.37 mmol) was added portion wise to a stirred solution of 1-ethynyl-3,5-dimethoxybenzene (5.00 g, 30.83 mmol) in MeCN (40 ml) at 0°C under nitrogen. Solution was allowed to warm slowly to ambient temperature and stirred for 18 h. Reaction mixture was quenched by addition of water and then partitioned between EtOAc and water. Organics were combined, dried, filtered and concentrated. Crude material was purified by FCC on Biotage (20 - 80% EtOAc / Heptane) to give the title compound (420.0 mg, 7%).
¹H NMR (500 MHz, Chloroform-*d*) δ 6.65 (t, *J*= 8.0 Hz, 1H), 3.88 (s, 6H), 3.52 (s, 1H). M/Z: 198.05, M+1: 198.85, t_{R}= 1.32 min (System 1).

### 5-(Benzenesulfonyl)-2-[2-(2,6-difluoro-3,5-dimethoxyphenyl)ethynyl]-5H-pyrrolo[2,3-b]pyrazine

5-(Benzenesulfonyl)-2-bromo-5H-pyrrolo[2,3-b]pyrazine (550.0 mg, 1.63 mmol), 3-ethynyl-2,4-difluoro-1,5-dimethoxybenzene (402.9 mg, 2.03 mmol), copper(1+) iodide (31.0 mg, 0.16 mmol) and DIPEA (1.41 ml, 0.01 mol) were dissolved in MeCN (10 ml). Reaction mixture degassed with nitrogen for 5 mins. Palladium tetrakis triphenylphospine (93.9 mg, 0.08 mmol) was added and reaction heated to 80°C in a sealed tube for 90 mins. Reaction mixture was cooled to RT, filtered and filtrate concentrated to dryness. Residue was dissolved in EtOAc, washed with water, dried, filtered and concentrated. Crude material was purified by FCC on Biotage (0 - 50% EtOAc / Heptane) to give the title compound as a yellow solid (252.0 mg, 34%).
¹H NMR (500 MHz, DMSO-*d*₆) δ 8.70 (s, 1H), 8.44 (d, *J=* 4.2 Hz, 1H), 8.14 (d, *J*= 7.5 Hz, 2H), 7.78 (t, *J* = 7.5 Hz, 1H), 7.66 (t, *J* = 7.9 Hz, 2H), 7.18 (t, *J* = 8.4 Hz, 1H), 7.08 (d, *J* = 4.2 Hz, 1H), 3.91 (s, 6H). M/Z: 455.08, M+1: 455.90, t_{R}= 1.54 min (System 1).

### 5-(benzenesulfonyl)-2-[2-(2,6-difluoro-3,5-dimethoxyphenyl)ethyl]-5H-pyrrolo[2,3-b]pyrazine

5-(benzenesulfonyl)-2-[2-(2,6-difluoro-3,5-dimethoxyphenyl)ethynyl]-5H-pyrrolo[2,3-b]pyrazine (252 mg, 0.55 mmol) was dissolved in EtOH:DMF (1:1 mixture) (4 mL), and Raney Nickel (0.36 µl, 0.06 mmol) (10 mol % slurry in water) added. The reaction mixture was placed under a hydrogen atmosphere and stirred at room temperature for five minutes. The reaction mixture was filtered through celite and washed with copious amounts of methanol. 1. The filtrate was concentrated and the DMF washed out by partitioning between water and EtOAc. The combined organics were concentrated and the crude mixture purified by FCC on Biotage (0-50 % EtOAc / Heptane) to afford the title compound as a white solid (81 mg, 32 %).
¹H NMR (CDCl₃, 500 MHz) δ 8.17 (1H, s), 8.15 (2H, s), 7.97 (1H, d, *J*=4.1 Hz), 7.61 (1H, t, *J*=7.5 Hz), 7.51 (2H, t, *J*=7.9 Hz), 6.81 (1H, d, *J*=4.1 Hz), 6.51 (1H, t, *J*=8.1 Hz), 3.85 (6H, s), 3.15 (4H, s). M/Z: 459.47, M+1: 460.05, t_{R}= 1.43 min (System 1).

### 5-(benzenesulfonyl)-2-[2-(2,6-difluoro-3,5-dimethoxyphenyl)ethyl]-6-iodo-5H-pyrrolo [2,3-b] pyrazine

5-(benzenesulfonyl)-2-[2-(2,6-difluoro-3,5-dimethoxyphenyl)ethyl]-5H-pyrrolo[2,3-b]pyrazine (81 mg, 0.18 mmol) was dissolved in THF (2 mL) and cooled to -78 °C. LDA (2 M in THF, 0.11 ml) was added and the reaction allowed to stir at -78 °C for 2 h. At this point a solution of iodine (58.17 mg, 0.23 mmol) in THF (2 mL) was added at - 78 °C and the reaction stirred at -78 °C for 5 min. The reaction mixture was quenched at -78 °C with saturated aq. NaHCO₃ solution and allowed to warm to RT. The reaction mixture was extracted into EtOAc and the combined organics were washed with 10 % Na₂S₂O₃ solution, dried, filtered and concentrated. The crude material was purified on Biotage (1-50% EtOAc / Heptane) to give the title compound as a 1:1 mixture with unreacted starting material (53 mg, 26 %)
¹H NMR (CDCl₃, 500 MHz) δ 8.17 (1H, s) 8.15 - 8.15 (2H, m), 8.08 (1H, s), 7.60 - 7.61 (1H, m), 7.51 - 7.52 (2H, m), 7.20 (1H, s), 6.51 (1H, s), 3.85 (6H, s), 3.17 - 3.29 (4H, m). M/Z: 485.36, M+1: 485.75, t_{R}= 1.55 min (System 1).

### 1-(4-{2-[2-(2,6-difluoro-3,5-dimethoxyphenyl)ethyl]-5H-pyrrolo[2,3-b]pyrazin-6-yl}phenyl)-4-methylpiperazine

5-(benzenesulfonyl)-2-[2-(2,6-difluoro-3,5-dimethoxyphenyl)ethyl]-6-iodo-5H-pyrrolo[2,3-b]pyrazine (50%, 53 mg, 0.05 mmol), 1-methyl-4-[4-(tetramethyl-1,3,2-dioxaborolan-2-yl)phenyl]piperazine (15.73 mg, 0.05 mmol) and potassium carbonate hydrate (4:2:3) (44.88 mg, 0.14 mmol) were dissolved in 1:1 dioxane:water (1 mL) and degassed with nitrogen for 5 mins. Palladium tetrakis triphenylphospine (5.23 mg, 0.005 mmol) was added and the reaction vessel sealed and heated to 100 °C for 2 h. Reaction mixture was cooled to RT and concentrated. Residue was diluted with water and extracted into EtOAc (x 3). Organics were combined, concentrated to 5 ml and loaded directly onto an SCX column. MeOH / NH₃ fractions were collected and concentrated to dryness. Resulting material was dissolved in MeOH (1 ml) and NaOH (5M in water, 1 ml) was added. Reaction was heated to 50°C for 4 h. Reaction mixture was cooled to RT, extracted into EtOAc (x 3) and washed with water. Crude material was purified by SCX column, followed by preparative basic HPLC to give the title compound as a yellow solid (3 mg, 13 %)
¹H NMR (CDCl₃, 250 MHz) δ 10.57 (1H, s), 7.96 (1H, s), 7.70 (2H, d, *J*=8.8 Hz), 7.05 (2H, d, *J*=8.9 Hz), 6.84 (1H, d, *J*=2.0 Hz), 6.51 (1H, t, *J*=8.1 Hz), 3.85 (6H, s), 3.31 - 3.41 (4H, m), 3.20 (4H, s), 2.58 - 2.68 (4H, m), 2.39 (3H, d, *J*=4.8 Hz), M/Z: 493.55, M+1: 494.25, t_{R}=4.36 min (system 2).

## Claims

1. A method for the preparation of a compound of formula (I) 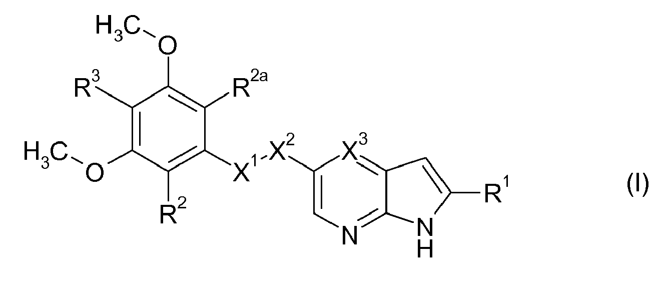 or a pharmaceutically acceptable salt thereof, wherein
X¹ is CH₂ and X² is CH₂; O; or N(R⁰), or
X¹ and X² are CH;
R⁰ is H; or C₁₋₄ alkyl, wherein C₁₋₄ alkyl is optionally substituted with one or more halogen, which are the same or different;
X³ is N; or CH;
R¹ is phenyl; naphthyl; 5 to 6 membered aromatic heterocyclyl; or 9 to 10 membered aromatic heterobicyclyl, wherein R¹ is optionally substituted with one or more R⁴, which are the same or different;
R⁴ is halogen; CN; C(O)OR⁵; OR⁵; C(O)R⁵; C(O)N(R⁵R^{5a}); S(O)₂N(R⁵R^{5a}); S(O)N(R⁵R^{5a}); S(O)₂R⁵; S(O)R⁵; N(R⁵)S(O)₂N(R^{5a}R^{5b}); SR⁵; N(R⁵R^{5a}); NO₂; OC(O)R⁵; N(R⁵)C(O)R^{5a}; N(R⁵)S(O)₂R^{5a}; N(R⁵)S(O)R^{5a}; N(R⁵)C(O)OR^{5a}; N(R⁵)C(O)N(R^{5a}R^{5b}); OC(O)N(R⁵R^{5a}); T¹; C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more R⁶, which are the same or different;
R⁵, R^{5a}, R^{5b} are independently selected from the group consisting of H; T¹; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more R⁶, which are the same or different;
R⁶ is halogen; CN; C(O)OR⁷; OR⁷; C(O)R⁷; C(O)N(R⁷R^{7a}); S(O)₂N(R⁷R^{7a}); S(O)N(R⁷R^{7a}); S(O)₂R⁷; S(O)R⁷; N(R⁷)S(O)₂N(R^{7a}R^{7b}); SR⁷; N(R⁷R^{7a}); NO₂; OC(O)R⁷; N(R⁷)C(O)R^{7a} ; N(R⁷)SO₂R^{7a}; N(R⁷)S(O)R^{7a}; N(R⁷)C(O)N(R^{7a}R^{7b}); N(R⁷)C(O)OR^{7a}; OC(O)N(R⁷R^{7a}); or T¹;
R⁷, R^{7a}, R^{7b} are independently selected from the group consisting of H; T¹; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
T¹ is phenyl; C₃₋₇ cycloalkyl; or 3 to 7 membered heterocyclyl, wherein T¹ is optionally substituted with one or more R⁸, which are the same or different;
R⁸ is halogen; CN; C(O)OR⁹; OR⁹; C(O)R⁹; C(O)N(R⁹R^{9a}); S(O)₂N(R⁹R^{9a}); S(O)N(R⁹R^{9a}); S(O)₂R⁹; S(O)R⁹; N(R⁹)S(O)₂N(R^{9a}R^{9b}); SR⁹; N(R⁹R^{9a}); NO₂; OC(O)R⁹; N(R⁹)C(O)R^{9a}; N(R⁹)S(O)₂R^{9a}; N(R⁹)S(O)R^{9a}; N(R⁹)C(O)OR^{9a}; N(R⁹)C(O)N(R^{9a}R^{9b}); OC(O)N(R⁹R^{9a}); oxo (=O), where the ring is at least partially saturated; C₁₋₆ alkyl; C₂₋₆ alkenyl; or C₂₋₆ alkynyl, wherein C₁₋₆ alkyl; C₂₋₆ alkenyl; and C₂₋₆ alkynyl are optionally substituted with one or more halogen, which are the same or different;
R⁹, R^{9a}, R^{9b} are independently selected from the group consisting of H; C₁₋₆ alkyl; C₂₋₆ alkenyl; and C₂₋₆ alkynyl, wherein C₁₋₆ alkyl; C₂₋₆ alkenyl; and C₂₋₆ alkynyl are optionally substituted with one or more halogen, which are the same or different;
R²; R^{2a}; R³ are independently selected from the group consisting of H; and halogen,
wherein the method comprises the step of
(a₀) reacting a compound of formula (Ia) 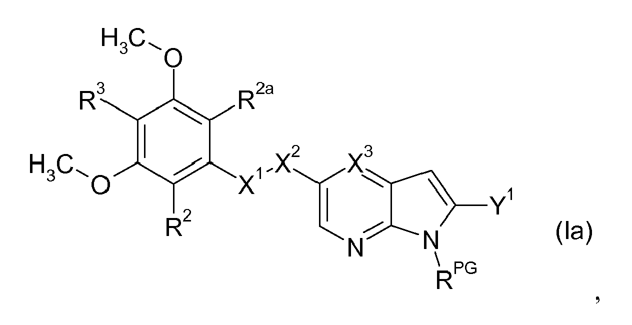 with a compound of formula R¹-Y² (Ib) in a coupling reaction, wherein X¹, X², X³, R², R^{2a}, R³ have the above meaning, R^{PG} is H or PG, wherein PG is a suitable protective group and Y¹, Y² are capable to react in a metal catalyzed coupling reaction forming carbon-carbon bond; and subsequent deprotection in order to remove PG, if present, to yield a compound of formula (I).

2. The method of claim 1, wherein Y¹ and Y² are selected in that
(i) Y¹ is B(OR^{A})₂ and Y² is Br, I or OS(O)₂CF₃; or Y¹ is Br, I and Y² is B(OR^{A})₂; or
(ii) Y¹ is SnR^{B}₃ and Y² is Br, I or OS(O)₂CF₃; or Y¹ is Br, I and Y² is SnR^{B}₃; or
(iii) Y¹ is ZnBr or ZnI and Y² is Br, I or OS(O)₂CF₃; or Y¹ is Br, I and Y² is ZnBr or ZnI,
wherein each R^{A} is independently H, C₁₋₄ alkyl or (OR^{A})₂ is pinacolate and each R^{B} is C₁₋₄ alkyl.

3. The method of claim 1 or 2, wherein PG is phenylsulfonyl or 4-methylphenylsulfonyl.

4. The method of any of claims 1 to 3, wherein Y¹ is I.

5. The method of any of claims 1 to 4, wherein Y² is B(OR^{A})₂.

6. The method of any of claims 1 to 5, wherein R¹ phenyl; or pyridyl, wherein R¹ is unsubstituted or substituted with one or more R⁴, which are the same or different.

7. The method of any of claims 1 to 6, wherein R³ is H.

8. The method of any of claims 1 to 7, wherein R², R^{2a} are independently selected from the group consisting of H; F; and Cl.

9. The method of any of claims 1 to 8, wherein the compound of formula (I) or a pharmaceutically acceptable salt thereof is selected from the group consisting of
1-((4-{2-[2-(3,5-dimethoxyphenyl)ethyl]-5H-pyrrolo[2,3-b]pyrazin-6-yl}phenyl)-4-methylpiperazine;
1-((4-{2-[(E)-2-(3,5-dimethoxyphenyl)ethenyl]5H-pyrrolo[2,3-b]pyrazin-6-yl} phenyl)4-methylpiperazine;
1-((4-{2-[2-(3,5-dimethoxyphenyl)ethyl]-5H-pyrrolo[2,3-b]pyrazine -6-yl}phenyl) piperazine;
1-((4-{2-[(E)-2-(3,5-dimethoxyphenyl)ethenyl]-5H-pyrrolo[2,3-b]pyrazin-6-yl} phenyl)piperazine;
N-[(3,5-dimethoxyphenyl)methyl]-6-[4-(4-methylpiperazin-1-yl)phenyl]-5H-pyrrolo [2,3-b]pyrazin-2-amine;
N-[(2,6-dichloro-3,5-dimethoxyphenyl)methyl]-6-[4-(4-methylpiperazin-1-yl) phenyl]-5H-pyrrolo[2,3-b]pyrazin-2-amine;
N-[(3,5-dimethoxyphenyl)methyl]-6-[4-(piperazin-1-yl)phenyl]-5H-pyrrolo[2,3-b] pyrazin-2-amine;
1-((4-{5-[(2,6-dichloro-3,5-dimethoxyphenyl)methoxy]-1H-pyrrolo[2,3-b]pyridin-2-yl}phenyl)-4-methylpiperazine;
N-[(2,6-dichloro-3,5-dimethoxyphenyl)methyl]-6-{5-[(4-methylpiperazin-1-yl) methyl]pyridin-2-yl}-5H-pyrrolo[2,3-b]pyrazin-2-amine;
N-[(2,6-dichloro-3,5-dimethoxyphenyl)methyl]-6-{5-[(4-ethylpiperazin-1-yl)methyl]pyridin-2-yl}-5H-pyrrolo[2,3-b]pyrazin-2-amine;
N-[(2,6-dichloro-3,5-dimethoxyphenyl)methyl]-6-[5-(morpholin-4-ylmethyl)pyridin-2-yl]-5H-pyrrolo[2,3-b]pyrazin-2-amine;
N-[(2,6-dichloro-3,5-dimethoxyphenyl)methyl]-6-[5-(piperazin-1-ylmethyl)pyridin-2-yl]-5H-pyrrolo[2,3-b]pyrazin-2-amine;
1-((4-{2-[2-(2,6-dichloro-3,5-dimethoxyphenyl)ethyl]-5H-pyrrolo[2,3-b]pyrazin-6-yl}phenyl)-4-methylpiperazine;
1-((4-{5-[2-(3,5-Dimethoxyphenyl)ethyl]-1H-pyrrolo[2,3-b]pyridin-2-yl}phenyl)-4-methylpiperazine; and
1-((4-{2-[2-(2,6-difluoro-3,5-dimethoxyphenyl)ethyl]-5H-pyrrolo[2,3-b]pyrazin-6-yl}phenyl)-4-methylpiperazine.

10. The method of any of claims 1 to 9, wherein Y¹ is I and wherein the method comprises before step (a₀) a step
(a₋₁) iodination of a compound of formula (II) 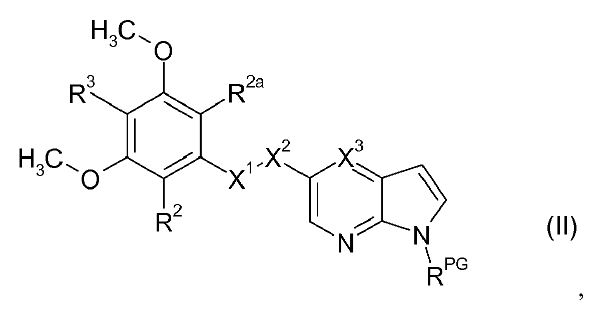 wherein R², R^{2a}, R³, X¹, X², X³ have the meaning as in any of claims 1 to 9 and R^{PG} is PG, resulting in a compound of (Ia) 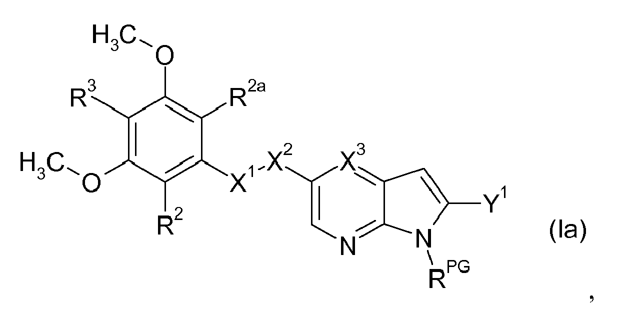 wherein R², R^{2a}, R³, X¹, X², X³ have the meaning as in any of claims 1 to 9, Y¹ is I and R^{PG} is PG.

11. The method of claim 10, wherein the iodination is carried out using iodine.

12. The method of claim 10 or 11, wherein between step (a₋₁) and (a₀) is a step of deprotecting a compound of formula (Ia), wherein R², R^{2a}, R³, X¹, X², X³ have the meaning as in any of claims 1 to 9, Y¹ is I and R^{PG} is PG, resulting in a compound of formula (Ia), wherein R², R^{2a}, R³, X¹, X², X³ have the meaning as in any of claims 1 to 9, Y¹ is I and R^{PG} is H.

13. The method of any of claims 10 to 12, wherein before step (a₀) a compound of formula (Ia), wherein R², R^{2a} are H; R³, X¹, X², X³ have the meaning as in any of claims 1 to 9; Y¹ is I and R^{PG} is H or PG, is chlorinated resulting in a compound of formula (Ia), wherein R², R^{2a} are Cl; R³, X¹, X², X³ have the meaning as in any of claims 1 to 9; Y¹ is I and R^{PG} is H or PG.

14. A compound of formula (Ia) as indicated in any of claims 1 to 13.

15. The compound of claim 14, wherein Y¹ is I.
